# EUROPEAN PATENT APPLICATION

(11) **EP 3 318 621 A1**
(43) Date of publication of application: **09.05.2018**
(21) Application number: 16821333.8
(22) Date of filing: 01.07.2016
(51) Int. Cl.: C11D 1/28, A61K 8/02, A61K 8/19, A61K 8/46, A61Q 5/02, A61Q 19/10, C11D 1/12, C11D 1/22, C11D 1/66, C11D 3/04, C11D 17/08

(54) **LIQUID DETERGENT**

(30) Priority: 03.07.2015 JP 2015134103
(71) Applicant: Lion Corporation, Tokyo 130-8644 (JP)
(72) Inventor: MORIGAKI Atsunori, Tokyo 130-8644 (JP); KUBOZONO Takayasu, Tokyo 130-8644 (JP); IWASA Yukiko, Tokyo 130-8644 (JP)
(74) Representative: Cabinet Laurent & Charras
(86) International application number: PCT/JP2016/069662
(87) International publication number: WO 2017/006864

(57) **Abstract**

A liquid detergent including: 3% by mass or more of an α-sulfo fatty acid alkyl ester salt (a) represented by the general formula (I); 5% by mass or more of at least one type of surfactant (b) selected from sulfonic acid-type anionic surfactants, sulfuric acid ester-type anionic surfactants and nonionic surfactants other than the component (a); and a water-soluble magnesium salt (c),
wherein the total content of the component (a) and the component (b) is from 8 to 35% by mass, the content of the component (a) is 50% by mass or less with respect to the total mass of the surfactant, and a mass ratio represented by the formula: (the component (c)) / (the component (a)) is from 0.1 to 1.5,

R¹-CH(SO₃M)-COOR² (I)

wherein R¹ is a hydrocarbon group having 14 to 16 carbon atoms, R² is a hydrocarbon group having 1 to 6 carbon atoms, and M is a counter ion.

## Description

### [Technical Field]

The present invention relates to a liquid detergent.

Priority is claimed on Japanese Patent Application No. 2015-134103, filed July 3, 2015, the content of which is incorporated herein by reference.

### [Background Art]

There are cases where high viscosity is required for a liquid detergent from the viewpoints of usability, palatability and the like.

Patent Document 1 discloses a liquid detergent having an increased viscosity by using at least one member selected from the group consisting of polyoxyethylene alkyl ether sulfates and alkylbenzene sulfonates in combination with an inorganic salt or the like.

### [Citation List]

### [Patent Document]

[Patent Document 1] International Patent Publication No. 2012/115250

### [Summary of Invention]

### [Technical Problem]

In recent years, detergents containing an α-sulfo fatty acid alkyl ester salt (hereinafter also referred to as "a-SF salt") as a detergent component have been widely used because of excellent detergency, favorable biodegradability and less environmental impact.

However, in the liquid detergent containing the α-SF salt, if an inorganic salt is added in order to increase the viscosity of the liquid detergent, when being stored under a low temperature environment, the liquid detergent solidifies or precipitates are formed to impair the liquid stability (in other words, the low temperature stability is impaired) in some cases. In particular, the low temperature stability tends to be impaired in a liquid detergent using an α-SF salt having a large number of carbon atoms in a fatty acid residue (a-SF salt in which the fatty acid residue has 16 or more carbon atoms).

In order to improve the low temperature stability of the liquid detergent, for example, it is conceivable to add a hydrotropic agent to the liquid detergent. However, in this case, the viscosity of the liquid detergent decreases, making it difficult to obtain a sufficient viscosity.

The present invention takes the above circumstances into consideration, with an object of providing a liquid detergent containing an α-SF salt and having an increased viscosity and excellent low temperature stability.

### [Solution to Problem]

As a result of intensive studies, the inventors of the present invention have found that the following liquid detergent can solve the above-mentioned problems.

That is, the present invention has the following constitutions.
[1] A liquid detergent including: a surfactant containing an α-sulfo fatty acid alkyl ester salt (a) represented by the following general formula (I) and
   at least one type of surfactant (b) selected from sulfonic acid-type anionic surfactants, sulfuric acid ester-type anionic surfactants and nonionic surfactants other than the aforementioned component (a); and
   a water-soluble magnesium salt (c), wherein
   the content of the aforementioned component (a) is 3% by mass or more with respect to the total mass of the liquid detergent,
   the content of the aforementioned component (b) is 5% by mass or more with respect to the total mass of the liquid detergent,
   the total content of the aforementioned component (a) and the aforementioned component (b) is from 8 to 35% by mass with respect to the total mass of the liquid detergent,
   the content of the aforementioned component (a) is 50% by mass or less with respect to the total mass of the surfactant, and
   a mass ratio represented by the formula: (the aforementioned component (c)) / (the aforementioned component (a)) is from 0.1 to 1.5,

   R¹-CH(SO₃M)-COOR² (I)

   in the formula (I), R¹ is a hydrocarbon group having 14 to 16 carbon atoms, R² is a hydrocarbon group having 1 to 6 carbon atoms, and M is a counter ion.
[2] The liquid detergent according to [1], wherein the aforementioned component (b) contains an alkylbenzene sulfonate (b1), and a mass ratio represented by the formula: (content of (b1)) / {(content of (b)) - (content of (b1))} is equal to or more than 1.
[3] The liquid detergent according to [1] or [2], wherein a viscosity at 25°C is equal to or more than 500 mPa·s.

### [Advantageous Effects of Invention]

According to the present invention, it is possible to provide a liquid detergent containing an α-SF salt and having an increased viscosity and excellent low temperature stability.

### [Description of Embodiments]

### (Liquid detergent)

A liquid detergent of the present invention is a liquid composition containing components (a) to (c).

### <Component (a)>

The component (a) is an α-sulfo fatty acid alkyl ester salt (a-SF salt) represented by the following general formula (I).

R¹-CH(SO₃M)-COOR² (I)

In the formula (I), R¹ is a hydrocarbon group having 14 to 16 carbon atoms, R² is a hydrocarbon group having 1 to 6 carbon atoms, and M is a counter ion.

In the above formula (I), the hydrocarbon group represented by R¹ may be linear or branched, or may contain a cyclic structure. In particular, the hydrocarbon group represented by R¹ is preferably an aliphatic hydrocarbon group, more preferably a linear or branched alkyl group or a linear or branched alkenyl group, and still more preferably a linear alkyl group or a linear alkenyl group. The number of carbon atoms of R¹ is from 14 to 16.

The hydrocarbon group represented by R² may be linear or branched, or may contain a cyclic structure when the number of carbon atoms is from 3 to 6. In particular, the hydrocarbon group represented by R² is preferably an aliphatic hydrocarbon group, more preferably a linear or branched alkyl group or a linear or branched alkenyl group, and still more preferably a linear alkyl group or a branched alkyl group. The number of carbon atoms of R² is from 1 to 6, and preferably from 1 to 3. Examples of the hydrocarbon group represented by R² include a methyl group, an ethyl group, an n-propyl group and an isopropyl group, and a methyl group, an ethyl group and an n-propyl group are preferable, and a methyl group is particularly preferable because detergency is further improved as a detergent component.

M is a counter ion and may be any ion as long as it can form a water-soluble salt with R¹CH(COOR²)SO₃⁻. Examples of the counter ion include an alkali metal ion, a protonated amine and ammonium. Examples of the alkali metal that can be the counter ion include sodium, potassium and the like. Examples of the amine that can be the counter ion include primary to tertiary amines. The total number of carbon atoms of the amine is preferably from 1 to 6. Further, the amine may have a hydroxy group. Since the solubility of the α-SF salt in water increases, it is preferable that the amine have a hydroxy group. Examples of the amine include alkanolamines, and the alkanol group preferably has 1 to 3 carbon atoms. Examples of the alkanolamine include monoethanolamine, diethanolamine and triethanolamine.

M is preferably an alkali metal ion, and particularly preferably a sodium ion, because it is easy to obtain and the low temperature stability of the liquid detergent is more easily enhanced.

Among the component (a), a compound in which R¹ in the above formula (I) is a linear or branched alkyl group, or a linear or branched alkenyl group, having 14 to 16 carbon atoms, and R² is a methyl group is particularly preferred.

Any one type of the component (a) may be used alone, or two or more types thereof may be used in combination.

As the component (a), it is preferable to use a mixture obtained by mixing fatty acid residues (referring to acyl group moieties) having different numbers of carbon atoms since the detergency as a detergent component increases and the solubility in water increases. More specifically, as the α-SF salt represented by the above general formula (I), those containing an α-SF salt (al) in which R¹ has 14 carbon atoms and an α-SF salt (a2) in which R¹ has 16 carbon atoms in a mass ratio of al: a2 = 45: 55 to 95: 5 are preferred, and the mass ratio is more preferably from 60: 40 to 90: 10, and still more preferably from 80: 20 to 85: 15. When the mass ratio falls within the above preferable range, the detergency, solubility in water, and appearance stability become better.

In the liquid detergent of the present invention, the content of the component (a) is 50% by mass or less with respect to the total mass of the surfactant. If the content of the component (a) exceeds 50% by mass with respect to the total mass of the surfactant, the low temperature stability is impaired.

The content of the component (a) is preferably less than 50% by mass and more preferably 40% by mass or less with respect to the total mass of the surfactant.

Further, the content of the component (a) is preferably 10% by mass or more, more preferably 15% by mass or more, and still more preferably 20% by mass or more, with respect to the total mass of the surfactant. When the content of the component (a) is equal to or more than the above lower limit value, the effect of the present invention obtained by using the component (a) and the components (b) to (c) described later in combination can be achieved more easily.

The content of the component (a) is preferably from 10 to 50% by mass, more preferably from 10% by mass or more to less than 50% by mass, still more preferably from 15% by mass or more to less than 50% by mass, and particularly preferably from 20 to 40% by mass with respect to the total mass of the surfactant.

Further, the content of the component (a) can be appropriately selected in the range of 3% by mass or more with respect to the total mass of the liquid detergent. The content of the component (a) is preferably 5% by mass or more with respect to the total mass of the liquid detergent, from the viewpoint that the effect of the present invention can be achieved more easily. Further, from the viewpoint that it is easy to obtain a liquid detergent more excellent in low temperature stability, the content of the component (a) is preferably 15% by mass or less, more preferably 10% by mass or less, and still more preferably 7% by mass or less, with respect to the total mass of the liquid detergent.

The content of the component (a) is preferably from 3 to 15% by mass, more preferably from 3 to 10% by mass and still more preferably from 5 to 7% by mass with respect to the total mass of the liquid detergent.

### <Component (b)>

The component (b) is at least one selected from sulfonic acid-type anionic surfactants, sulfuric acid ester-type anionic surfactants and nonionic surfactants, other than the component (a). Here, the sulfonic acid-type anionic surfactant is an anionic surfactant having a -SO₃⁻M⁺ group in the molecule. The sulfuric acid ester-type anionic surfactant is an anionic surfactant having a -OSO₃⁻M⁺ group in the molecule. Here, M⁺ is a counter cation.

In the present invention, by using the component (b) and the component (c) described later in combination in the liquid detergent containing the component (a), the viscosity is increased and the low temperature stability is improved.

Examples of the sulfonic acid-type anionic surfactant other than the component (a) include linear alkylbenzene sulfonates (LAS), α-olefin sulfonates (AOS), alkanesulfonates, and α-SF salts and the like in which fatty acid residues have 8 to 14 carbon atoms.

As the linear alkylbenzene sulfonate, those in which a linear alkyl group has 8 to 20 carbon atoms are preferred, and those in which a linear alkyl group has 10 to 14 carbon atoms are more preferred.

As the α-olefin sulfonate, those having 10 to 20 carbon atoms are preferred, and those having 10 to 16 carbon atoms are more preferred.

As the alkanesulfonate, a secondary alkanesulfonate having an alkyl group of 10 to 20 carbon atoms, preferably 14 to 18 carbon atoms, is preferred.

As the sulfuric acid ester-type anionic surfactant, alkyl sulfates, alkyl ether sulfates or alkenyl ether sulfates (AES) and the like can be mentioned. As the alkyl sulfate, those that are linear or branched and having 10 to 20 carbon atoms are preferred, and those that are linear or branched and having 10 to 16 carbon atoms are more preferred.

The alkyl ether sulfate is preferably a polyoxyalkylene alkyl ether sulfate represented by the following formula (II).

R⁴⁰-O-[(EO)ₘ/(PO)ₙ]-SO₃⁻M⁺ (II)

[In the formula (II), R⁴⁰ is a linear or branched alkyl group having 8 to 20 carbon atoms. EO represents an oxyethylene group, and PO represents an oxypropylene group. m represents the average number of repetitions of EO and is a number of 1 or more. n represents the average number of repetitions of PO and is a number of 0 to 6. M⁺ is a counter cation. When n is greater than 0, PO and EO in [(EO)ₘ/(PO)ₙ] may be arranged in a block form or a random form. Further, PO may bind to "R⁴⁰-O- " and EO may bind to "R⁴⁰-O-".]

As the polyoxyalkylene alkyl ether sulfate, those having a linear or branched alkyl group of 10 to 20 carbon atoms and having an average of 1 to 10 moles of alkylene oxide added thereto are preferable.

The number of carbon atoms of the alkyl group is preferably from 10 to 20 and more preferably from 12 to 14. Specific examples thereof include a dodecyl group, a tridecyl group and a tetradecyl group.

The average number of repetitions of EO is preferably from 1 to 10 and more preferably from 2 to 7.

The average number of repetitions of PO is preferably from 0 to 3.

The alkyl ether sulfate is preferably a polyoxyethylene alkyl ether sulfate having a linear or branched alkyl group of 10 to 20 carbon atoms and having an average of 1 to 10 moles of ethylene oxide added thereto. In particular, from the viewpoint of improving the liquid stability of the liquid detergent, the number of carbon atoms of the alkyl group of the polyoxyethylene alkyl ether sulfate is more preferably from 10 to 14, and from the viewpoint of improving the detergency, the average number of moles of ethylene oxide added is more preferably from 1 to 4.

The alkenyl ether sulfate is preferably a polyoxyethylene alkenyl ether sulfate having a linear or branched alkenyl group of 10 to 20 carbon atoms and having an average of 1 to 10 moles of ethylene oxide added thereto.

Examples of salts of sulfonic acid-type anionic surfactant and sulfuric acid ester-type anionic surfactant other than the above component (a) include salts of alkali metals such as sodium and potassium, alkanolamine salts such as monoethanolamine salts, diethanolamine salts and triethanolamine salts, alkylamine salts and ammonium salts. Among them, an alkali metal salt is preferable.

Examples of the nonionic surfactant include a polyoxyalkylene-type nonionic surfactant obtained by adding an alkylene oxide to a higher alcohol, an alkylphenol, a higher fatty acid, a higher fatty acid ester, a higher amine or the like, a polyoxyethylene polyoxypropylene block copolymer, a fatty acid alkanolamide, a polyhydric alcohol fatty acid ester or an alkylene oxide adduct thereof, a fatty acid polyglycerol ester, a sugar fatty acid ester, an alkyl (or alkenyl) amine oxide, an amidoamine oxide, an alkylene oxide adduct of hydrogenated castor oil, an N-alkyl polyhydroxy fatty acid amide, an alkyl glycoside, an alkyl polyglycoside and a glyceryl ether. It should be noted that the term "higher" as used herein means a compound having 8 or more carbon atoms.

As the nonionic surfactant, a polyoxyalkylene-type nonionic surfactant is preferable. Among the polyoxyalkylene-type nonionic surfactants, a polyoxyalkylene alkyl ether (AE) in which an alkylene oxide is added to a saturated higher alcohol is preferred. As the polyoxyalkylene alkyl ether, those represented by the following formula (III) are preferred.

R⁴-O-[(EO)ᵥ/(PO)_{w}]-(EO)ₓ-H (III)

(In the formula (III), R⁴ is a hydrocarbon having 6 to 22 carbon atoms, v represents an average number of repetitions of EO and is a number of 3 to 20, w represents an average number of repetitions of PO and is a number of 0 to 6, x represents an average number of repetitions of EO and is a number of 0 to 20, EO represents an oxyethylene group, and PO represents an oxypropylene group.)

The number of carbon atoms of the alkyl group of the polyoxyalkylene alkyl ether is preferably from 8 to 18, and the number of carbon atoms of the alkyl group is more preferably from 10 to 16, and particularly preferably from 10 to 14 from the viewpoint of improving the detergency. When the number of carbon atoms of the alkyl group is 8 or more, the function as a surfactant can be sufficiently exhibited, and excellent detergency can be imparted to the liquid detergent. On the other hand, when the number of carbon atoms of the alkyl group is 18 or less, because the liquid state is easily maintained, the liquid stability of the liquid detergent is further improved.

The alkylene oxide may be any one of ethylene oxide, propylene oxide, and butylene oxide, or may be a mixture of two or more of them. Among the alkylene oxides, ethylene oxide and propylene oxide are preferable, and ethylene oxide is more preferable. In the case where two or more kinds of ethylene oxide, propylene oxide or butylene oxide are mixed, they may be mixed at random or mixed in a block form. The average number of moles of alkylene oxide added is a number of 5 to 30, and is preferably a number of 5 to 20 and more preferably a number of 5 to 10 from the viewpoint of improving the stability of the liquid detergent.

Any one type of the component (b) may be used alone, or two or more types thereof may be used in combination.

As the component (b), a linear alkylbenzene sulfonate (b1), a polyoxyethylene alkyl ether sulfate (b2) and a polyoxyalkylene alkyl ether (b3) are preferred.

From the viewpoint that it is easy to obtain a liquid detergent having better low temperature stability, it is preferable that the component (b) contain the component (b1). Further, as the component (b), it is preferable that the components (b1) to (b3) are used in combination.

The content of the component (b) is equal to or more than 5% by mass with respect to the total mass of the liquid detergent. The content of the component (b) is preferably from 5 to 32% by mass, more preferably from 7 to 25% by mass, still more preferably from 7 to 20% by mass, and particularly preferably from 10 to 20% by mass with respect to the total mass of the liquid detergent.

When the content of the component (b) is within the above-mentioned preferred range, it becomes easy to obtain a liquid detergent more excellent in low temperature stability.

The total content of the component (a) and the component (b) in the liquid detergent [hereinafter also referred to as "total content of (a + b)"] is from 8 to 35% by mass with respect to the total mass of the liquid detergent. If the total content of (a + b) is less than 8% by mass or more than 35% by mass, the viscosity cannot be sufficiently increased or the low temperature stability may be impaired.

The total content of (a + b) is preferably from 10 to 25% by mass from the viewpoints of enhancing the viscosity and easily obtaining a liquid detergent excellent in low temperature stability.

Further, the total content of the component (a) and the component (b) with respect to the total mass of the surfactant is preferably 50% by mass, more preferably 75% by mass or more, still more preferably 80% by mass or more, particularly preferably 90% by mass or more, and may be 100% by mass.

When the total content of the component (a) and the component (b) with respect to the total mass of the surfactant is equal to or more than the above-mentioned lower limit value, it becomes easy to obtain a liquid detergent having enhanced viscosity and excellent low temperature stability.

A mass ratio represented by the formula: (content of (b1)) /{(content of (b)) - (content of (b1))} [i.e., a mass ratio of the content of the component (b1) with respect to the total content of components other than the component (b1) in the component (b), hereinafter also referred to as "bl / (b - b1) ratio"] is preferably 1 or more, and more preferably 1.5 or more.

When the b1 / (b - b1) ratio is equal to or more than the above lower limit value, the low temperature stability is further enhanced.

The b1 / (b - b1) ratio is preferably from 1.0 to 5.0, and more preferably from 1.5 to 4.0.

### <Component (c)>

The component (c) is a water-soluble magnesium salt. In the present invention, by using the component (b) and the component (c) in combination in the liquid detergent containing the component (a), the viscosity is increased and the low temperature stability is improved.

As the component (c), a water-soluble inorganic salt is preferable. It should be noted that in the present invention, the term "water-soluble" means that 5% by mass or more is dissolved in water of 25°C.

As the component (c), for example, sulfates and chlorides of magnesium and the like can be mentioned. As the component (c), hydrates of the above-mentioned sulfates, chlorides or the like may be used.

As the component (c), magnesium sulfate and magnesium chloride are preferable, and magnesium sulfate is more preferable from the viewpoints of higher viscosity and low temperature stability.

Any one type of the component (c) may be used alone, or two or more types thereof may be used in combination.

The content of the component (c) is preferably from 0.1 to 12% by mass, more preferably from 0.5 to 10% by mass, still more preferably from 0.5 to 5% by mass, and particularly preferably from 1 to 3% by mass with respect to the total mass of the liquid detergent. When the content of the component (c) is within the above-mentioned preferred range, it becomes easy to obtain a liquid detergent having enhanced viscosity and excellent low temperature stability.

A mass ratio represented by the formula: (component (c)) / (component (a)) [mass ratio of the content of the component (c) with respect to the content of the component (a), hereinafter also referred to as "c / a ratio"] is from 0.1 to 1.5.

When the c / a ratio is 0.1 or more, the viscosity is easily increased sufficiently. Further, the low temperature stability is impaired. When the c / a ratio is 1.5 or less, the low temperature stability is easily enhanced.

The c / a ratio is more preferably from 0.1 to 1.0, and still more preferably from 0.1 to 0.8.

A mass ratio represented by the formula: (component (c)) / (component (b)) [mass ratio of the content of the component (c) with respect to the content of the component (b), hereinafter also referred to as "c / b ratio"] is preferably from 7 to 40.

### <Water>

The liquid detergent of the present invention preferably contains water from the viewpoints of ease of handling during production, solubility in water at the time of use, and the like.

The content of water in the liquid detergent is not particularly limited, but is preferably from 50 to 92% by mass and more preferably from 60 to 85% by mass with respect to the total mass of the liquid detergent.

### <Other components>

In addition to the above components (a) to (c), the liquid detergent of the present invention may contain other components usually used in a liquid detergent.

Examples of other components include a surfactant other than the components (a) and (b), a water-miscible organic solvent, a chelating agent, a bactericide, an antiseptic agent, an antifungal agent, a pigment, an antioxidant, an ultraviolet absorber, a perfume and a pH adjusting agent.

Examples of surfactants other than the above components (a) and (b) include carboxylic acid-type or phosphoric acid-type anionic surfactants, amphoteric surfactants and the like.

Examples of the above carboxylic acid-type anionic surfactant include higher fatty acid salts (soaps) having 8 to 24 carbon atoms, alkyl ether carboxylates, polyoxyalkylene ether carboxylates, alkyl (or alkenyl) amide ether carboxylates and acylaminocarboxylates.

Examples of the above phosphoric acid-type anionic surfactant include alkyl phosphates, polyoxyalkylene alkyl phosphates, polyoxyalkylene alkylphenyl phosphates and glycerin fatty acid ester monophosphates.

Examples of these salts include salts of alkali metals such as sodium and potassium; alkanolamine salts such as monoethanolamine salts, diethanolamine salts and triethanolamine salts; and ammonium salts. Among them, an alkali metal salt is preferable.

As the amphoteric surfactant, amphoteric surfactants of alkyl betaine-type, alkyl amide betaine-type, imidazoline-type, alkyl amino sulfonic acid-type, alkyl amino carboxylic acid-type, alkyl amide carboxylic acid-type, amide amino acid-type, phosphoric acid-type and the like can be mentioned.

In the liquid detergent of the present invention, the total content of the surfactant is preferably 50% by mass or less, more preferably 35% by mass or less, and still more preferably 25% by mass or less with respect to the total mass of the liquid detergent. Further, the total content of the surfactant is preferably 8% by mass or more and more preferably 10% by mass or more with respect to the total mass of the liquid detergent. More specifically, the total content of the surfactant is preferably from 8 to 50% by mass, more preferably from 8 to 35% by mass, and still more preferably from 10 to 25% by mass with respect to the total mass of the liquid detergent.

When the total content of the surfactant is in the above-mentioned preferable range, it becomes easy to obtain a liquid detergent having enhanced viscosity and excellent low temperature stability.

Examples of the water-miscible organic solvent include alcohols such as ethanol, 1-propanol, 2-propanol and 1-butanol, glycols such as propylene glycol, butylene glycol and hexylene glycol, polyglycols such as diethylene glycol, triethylene glycol, tetraethylene glycol, polyethylene glycol and dipropylene glycol having a weight average molecular weight of about 200 to 1,000, and alkyl ethers such as diethylene glycol monomethyl ether, diethylene glycol monoethyl ether, diethylene glycol monobutyl ether and diethylene glycol dimethyl ether.

It should be noted that in the present invention, the water-miscible organic solvent refers to an organic solvent that dissolves in an amount of 50 g or more in 1 L of deionized water at 25°C.

A pH adjusting agent may be added to the liquid detergent of the present invention in order to adjust the pH to a desired value.

However, when the pH of the liquid detergent becomes the desired value only by blending the above-mentioned components, the pH adjusting agent may not necessarily be added. Examples of the pH adjusting agent include acidic compounds such as sulfuric acid and hydrochloric acid, and alkaline compounds such as sodium hydroxide and potassium hydroxide. As the alkaline compound, amines other than the aforementioned alkanolamines can also be used. One type of these pH adjusting agents may be used alone or two or more types thereof may be used in combination.

The sum of the components (a) to (c) and water does not exceed 100% by mass.

The liquid detergent of the present invention preferably has a pH of 5 to 9 at 25°C, and more preferably has a pH of 7 to 9. If the pH of the liquid detergent is within the above-mentioned preferable range, when the liquid detergent is stored for a long time, since the α-SF salt is more stabilized, favorable detergency is easily maintained. In addition, by setting the pH to the above upper limit or less, the low temperature stability is more easily enhanced, which is preferable.

It should be noted that the pH of the liquid detergent at 25°C in the present invention is a value measured with a pH meter or the like (for example, using the "HM-30G" (product name) manufactured by DKK-TOA Corporation) after adjusting the sample to 25°C.

The viscosity of the liquid detergent of the present invention at 25°C is preferably equal to or more than 500 mPa·s, and more preferably equal to or more than 1,000 mPa·s. Further, the viscosity of the liquid detergent of the present invention at 25°C is preferably equal to or less than 20,000 mPa·s, and more preferably equal to or less than 10,000 mPa·s.

When the viscosity of the liquid detergent at 25°C is equal to or more than the above lower limit value, it can be clearly perceived that the viscosity of the liquid detergent has been increased and the palatability is enhanced. When the viscosity of the liquid detergent at 25°C is equal to or less than the above upper limit value, for example, it becomes easy to pour out the liquid detergent from a container and the usability is improved.

The viscosity of the liquid detergent at 25°C is preferably from 500 to 20,000 mPa·s, more preferably from 500 to 10,000 mPa·s, still more preferably from 500 to 5,000 mPa·s, particularly preferably from 750 to 4,000 mPa·s, and most preferably from 1,000 to 3,000 mPa·s.

It should be noted that the viscosity of the liquid detergent in the present invention at 25°C is a value measured using a B-type viscometer (manufactured by TOKIMEC Inc.) after adjusting the sample to 25°C.

The viscosity of the liquid detergent is a value obtained by rotating a rotor in a condition of 30 rpm and measured 30 seconds after the start of the rotation. The rotors to be used for the measurements are the rotors No. 1 to 2 (when the viscosity of the liquid detergent is less than 100 mPa·s), the rotor No. 3 (when the viscosity of the liquid detergent is from 100 to 5,000 mPa·s) and the rotor No. 4 (when the viscosity of the liquid detergent exceeds 5,000 mPa·s).

### (Method for producing liquid detergent)

The liquid detergent of the present invention can be produced, for example, by dissolving the above-mentioned components (a) to (c) and, if necessary, optional components in water.

The liquid detergent of the present invention can be used, for example, as a liquid detergent for textile products for cleaning garments or the like, a liquid detergent for kitchen for cleaning tableware, vegetables or the like, a liquid detergent for hard surfaces for cleaning toilet bowls, walls, bathrooms or the like, and a liquid detergent for human body such as body soap and shampoo for cleaning skin, hair or the like. In particular, it is suitably used as a liquid detergent for textile products.

As described above, since the liquid detergent of the present invention contains the components (a) to (c), and the total content of (a + b), the content of the component (a) with respect to the total mass of the surfactant, and the c / a ratio are within specific ranges, the viscosity is increased and the low temperature stability is excellent.

The liquid detergent of the present invention may include, for example, the following aspects.
[1] A liquid detergent including: 3% by mass or more of an α-sulfo fatty acid alkyl ester salt (a) represented by the following general formula (I) and
   5 mass% or more of at least one type of surfactant (b) selected from sulfonic acid-type anionic surfactants, sulfuric acid ester-type anionic surfactants and nonionic surfactants other than the aforementioned component (a); and
   a water-soluble magnesium salt (c), wherein
   the total content of the aforementioned component (a) and the aforementioned component (b) is from 8 to 35% by mass,
   the content of the aforementioned component (a) is 50% by mass or less with respect to the total mass of the surfactant, and
   a mass ratio represented by the formula: (the aforementioned component (c)) / (the aforementioned component (a)) is from 0.1 to 1.5,

   R¹-CH(SO₃M)-COOR² (I)

   in the formula (I), R¹ is a hydrocarbon group having 14 to 16 carbon atoms, R² is a hydrocarbon group having 1 to 6 carbon atoms, and M is a counter ion.
[2] The liquid detergent according to [1], wherein the aforementioned component (b) contains an alkylbenzene sulfonate (b1) and a mass ratio represented by the formula: (content of (b1)) / {(content of (b)) - (content of (b1))} is equal to or more than 1.
[3] The liquid detergent according to [1] or [2], wherein the aforementioned component (b) contains an alkylbenzene sulfonate (b1) and at least one surfactant selected from the group consisting of a polyoxyethylene alkyl ether sulfate (b2) and a polyoxyalkylene alkyl ether (b3).
[4] The liquid detergent according to any one of [1] to [3], wherein the total content of the aforementioned component (a) and the aforementioned component (b) is equal to or more than 50% by mass with respect to the total mass of the surfactant.
[5] The liquid detergent according to any one of [1] to [4], wherein a viscosity at 25°C is equal to or more than 500 mPa·s.
[6] The liquid detergent according to any one of [1] to [5], wherein a mass ratio of an α-sulfo fatty acid alkyl ester salt (al) in which the number of carbon atoms of R¹ in the formula (I) is 14 and an α-sulfo fatty acid alkyl ester salt (a2) in which the number of carbon atoms of R¹ in the formula (I) is 16, expressed by (al): (a2), is from 45: 55 to 95: 5.
[7] The liquid detergent according to any one of [1] to [6], wherein the aforementioned component (b) includes an alkylbenzene sulfonate, a polyoxyethylene alkyl ether sulfate represented by the above formula (II), and a polyoxyalkylene alkyl ether represented by the formula (III).
[8] The liquid detergent according to any one of [1] to [7], wherein the aforementioned component (c) includes at least one selected from the group consisting of magnesium sulfate and magnesium chloride.
[9] The liquid detergent according to any one of [1] to [8], further including water.

### [Examples]

A more detailed description of the present invention is presented below using a series of examples, although the present invention is in no way limited by these examples. In the examples, unless stated otherwise, the units "%" represent "% by mass".

The raw materials used in the present examples are as follows.

### <Component (a)>

a-1: MES salt (sodium salt of a sulfonated product of a mixture of methyl palmitate / methyl stearate = 85/15 in terms of mass ratio). In the formula (I), R¹ is a mixture where (a linear alkyl group having 14 carbon atoms): (a linear alkyl group having 16 carbon atoms) is 85: 15, R² = methyl group, M = sodium), which was synthesized by the following synthesis method.
α-2: MES salt (sodium salt of a sulfonated product of a mixture of methyl palmitate / methyl stearate = 60/40 in terms of mass ratio). In the formula (I), R¹ is a mixture where (a linear alkyl group having 14 carbon atoms): (a linear alkyl group having 16 carbon atoms) is 60: 40, R² = methyl group, M = sodium) which was synthesized by the following synthesis method.

### <Component (b)>

bl-1: LAS, sodium linear alkyl (10 to 14 carbon atoms) benzenesulfonate (sodium hydroxide neutralized product of "Lipon LH-200" (product name), manufactured by Lion Corporation).
b2-1: AES, sodium polyoxyethylene alkyl (12 to 14 carbon atoms) ether sulfate [average number of moles of ethylene oxide added: 2]. In the formula (II), R⁴⁰ = an alkyl group having 12 to 14 carbon atoms, m = 2, n = 0, M⁺ = sodium ion, "EMAL 270 N" (product name) manufactured by Kao Corporation.
b3-1: AE, polyoxyethylene alkyl (12 to 14 carbon atoms) ether (average number of moles of ethylene oxide added: 7), in the formula (III), R⁴ = an alkyl group having 12 to 14 carbon atoms, v = 7, w = 0, x = 0, "LEOX CL-70" (product name) manufactured by Lion Corporation.

### <Component (c)>

c-1: magnesium sulfate, reagent, manufactured by Tokyo Chemical Industry Co., Ltd.
c-2: magnesium chloride hexahydrate, reagent, manufactured by Wako Pure Chemical Industries, Ltd.

### <Component (c'): comparative component of the component (c)>

c'-1: sodium sulfate, reagent, manufactured by Tokyo Chemical Industry Co., Ltd.

### <Optional component>

Water: purified water.

### [Synthesis of a-1]

Methyl palmitate (Pastell M-16 (product name), manufactured by Lion Corporation) and methyl stearate (Pastel1 M-180 (product name), manufactured by Lion Corporation) were mixed so as to achieve a mass ratio of 85: 15 to obtain a fatty acid methyl ester mixture. 330 kg of this fatty acid methyl ester mixture was poured into a reaction apparatus equipped with a stirrer and having a capacity of 1 kL, and then 115.6 kg (1.2 times moles with respect to the fatty acid methyl ester mixture) of SO₃ gas (sulfonation gas) diluted to 4 vol% with nitrogen gas was bubbled while stirring the mixture. The reaction temperature was 80°C. The sulfonation gas was blown into the fatty acid methyl ester mixture at a constant rate over the course of 3 hours. Thereafter, anhydrous sodium sulfate was added in an amount of 1.5 parts by mass with respect to 100 parts by mass of the fatty acid methyl ester mixture, and the resulting mixture was aged for 30 minutes while being kept at 80°C.

Thereafter, 13.5 kg of methanol was supplied and esterification was carried out at a temperature condition of 80°C and an aging time of 30 minutes. Subsequently, the esterified product extracted from the reaction apparatus was continuously neutralized by adding an equivalent amount of aqueous sodium hydroxide solution using a line mixer. This neutralized product was poured into a bleaching agent mixing line, a 35 vol% hydrogen peroxide solution was supplied and mixed, and bleaching was conducted while maintaining the temperature at 80°C to obtain a-1 in the form of a paste.

### [Synthesis of a-2]

Methyl palmitate (Pastell M-16 (product name), manufactured by Lion Corporation) and methyl stearate (Pastel1 M-180 (product name), manufactured by Lion Corporation) were mixed so as to achieve a mass ratio of 6: 4 to obtain a fatty acid methyl ester mixture. 330 kg of this fatty acid methyl ester mixture was poured into a reaction apparatus equipped with a stirrer and having a capacity of 1 kL, and then anhydrous sodium sulfate as a coloring inhibitor was added in an amount of 5 parts by mass with respect to 100 parts by mass of the fatty acid methyl ester mixture while stirring the mixture. Thereafter, 112.8 kg (1.2 times moles with respect to the fatty acid methyl ester mixture) of SO₃ gas (sulfonation gas) diluted to 4 vol% with nitrogen gas was bubbled while continuing the stirring. The reaction temperature was 80°C. The sulfonation gas was blown into the fatty acid methyl ester mixture at a constant rate over the course of 3 hours. Thereafter, aging was carried out for 30 minutes while maintaining the temperature at 80°C.

Subsequently, esterification, neutralization and bleaching were carried out in the same manner as that described for a-1 to obtain a-2 in the form of a paste.

### <Examples 1 to 16, Comparative Examples 1 to 4>

In accordance with the compositions shown in Tables 1 and 2, the components (a) to (c) were added to water and mixed to obtain liquid detergents of Examples 1 to 16 and Comparative Example 4. Further, liquid detergents of Comparative Examples 1 and 3 were obtained in the same manner as described above, except that the component (b) or the component (c) was not added. A liquid detergent of Comparative Example 2 was obtained in the same manner as described above, except that the component (c') was used in place of the component (c).

Tables 1 and 2 show the compositions (ingredients, contents (% by mass)) of the obtained liquid detergents of each example.

In the tables, if there is a blank column for an ingredient, the ingredient was not added.

In the tables, the content of the ingredient indicates the amount in terms of pure content.

The term "balance" indicating the content of purified water refers to the amount of the remainder to be added so that the total amount (% by mass) of all the ingredients contained in the liquid detergent becomes 100% by mass.

In the table, the "c / a ratio" of Comparative Example 2 indicates a mass ratio of the component (c') with respect to the component (a).

For the liquid detergents of each example, viscosity and low temperature stability were evaluated as follows. The evaluation results are shown in Tables 1 and 2.

### [Evaluation of viscosity]

The viscosity of the liquid detergent of each example was measured by the above method. In addition, the measured values of viscosity were classified and evaluated according to the following criteria. The measured values of viscosity and the evaluation results are shown in Tables 1 and 2.

It should be noted that the liquid detergent evaluated as A was excellent in usability, and it was also possible to clearly perceive that the viscosity of the liquid detergent has been increased. Either the liquid detergent evaluated as B was somewhat excellent in usability (more than 10,000 mPa·s and not more than 20,000 mPa·s), or it was possible to perceive that the viscosity of the liquid detergent has been increased (500 mPa·s or more and less than 1,000 mPa·s). Either the liquid detergent evaluated as C was poor in usability (more than 20,000 mPa·s), or it was not possible to clearly perceive that the viscosity of the liquid detergent has been increased (less than 500 mPa·s).

### (Criteria)

A: 1,000 to 10,000 mPa·s.
B: 500 mPa·s or more to less than 1,000 mPa·s, or more than 10,000 mPa·s and not more than 20,000 mPa·s.
C: less than 500 mPa·s, or more than 20,000 mPa·s.

### [Evaluation of low temperature stability]

50 mL of the liquid detergent of each example was added to a colorless and transparent sample bottle, and the lid was closed and sealed. In this state, the sample bottles were left to stand in constant temperature baths at 5°C, 15°C and 20°C, respectively, and stored for 1 month. Thereafter, the appearance of the liquid was visually observed, and the low temperature stability of the liquid detergent was evaluated based on the following criteria.

### (Criteria)

A: At a storage temperature of 5°C, solidification of the liquid detergent does not occur, and no precipitate is observed in the liquid detergent.
B: At a storage temperature of 5°C, solidification of the liquid detergent occurs, or a precipitate is observed in the liquid detergent, but at a storage temperature of 15°C, solidification of the liquid detergent does not occur, and no precipitate is observed in the liquid detergent.
C: At a storage temperature of 15°C, solidification of the liquid detergent occurs, or a precipitate is observed in the liquid detergent, but at a storage temperature of 20°C, solidification of the liquid detergent does not occur, and no precipitate is observed in the liquid detergent.
D: At a storage temperature of 20°C, solidification of the liquid detergent occurs, or a precipitate is observed in the liquid detergent.

**[Table 1]**

| | | | Examples | | | | | | | | | | | | | | | |
|---|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 13 | 14 | 15 | 16 |
| Composition (% by mass) | Component (a) | a-1 | 7.0 | 7.0 | 7.0 | 7.0 | 7.0 | 7.0 | 7.0 | 10 | 10 | | | | | | | |
| | | a-2 | | | | | | | | | | 7.0 | 7.0 | 7.0 | 7.0 | 7.0 | 7.0 | 7.0 |
| | Component (b1) | b1-1 | 7.8 | 7.8 | 7.8 | 2.6 | | 7.8 | 10.4 | 8.0 | 16 | 7.8 | 7.8 | 7.8 | 2.6 | | 7.8 | 10.4 |
| | Component (b2) | b2-1 | 2.6 | 2.6 | 2.6 | 5.2 | 13 | 2.6 | | 2.0 | 4.0 | 3.6 | 2.6 | 2.6 | 5.2 | 13 | 2.6 | |
| | Component (b3) | b3-1 | 2.6 | 2.6 | 2.6 | 5.2 | | 2.6 | 2.6 | | | 2.6 | 2.6 | 2.6 | 5.2 | | 2.6 | 2.6 |
| | Component (c) | c-1 | 1.0 | 3.0 | 5.0 | 3.0 | 3.0 | | 3.0 | 3.0 | 1.3 | 1.0 | 3.0 | 5.0 | 5.0 | 3.0 | | 3.0 |
| | | c-2 | | | | | | 3.0 | | | | | | | | | 3.0 | |
| | Purified water | | Balance | Balance | Balance | Balance | Balance | Balance | Balance | Balance | Balance | Balance | Balance | Balance | Balance | Balance | Balance | Balance |
| | Total | | 100 | 100 | 100 | 100 | 100 | 100 | 100 | 100 | 100 | 100 | 100 | 100 | 100 | 100 | 100 | 100 |
| Total content of (a + b) (% by mass) | | | 20 | 20 | 20 | 20 | 20 | 20 | 20 | 20 | 20 | 20 | 20 | 20 | 20 | 20 | 20 | 20 |
| Content of component (a) with respect to total mass of surfactant (% by mass) | | | 35 | 35 | 35 | 35 | 35 | 35 | 35 | 50 | 33 | 35 | 35 | 35 | 35 | 35 | 35 | 35 |
| c/a ratio | | | 0.14 | 0.43 | 0.71 | 0.43 | 0.43 | 0.43 | 0.43 | 0.3 | 0.15 | 0.14 | 0.43 | 0.71 | 0.71 | 0.43 | 0.43 | 0.43 |
| b1 / (b - b1) ratio | | | 1.5 | 1.5 | 1.5 | 0.25 | 0 | 1.5 | 4.0 | 4.0 | 4.0 | 1.5 | 1.5 | 1.5 | 0.25 | 0 | 1.5 | 4.0 |
| Evaluation | Viscosity | Measured value (mPa· s, 25°C) | 866 | 2017 | 923 | 3820 | 16400 | 760 | 1060 | 1540 | 14700 | 840 | 2005 | 908 | 3750 | 15900 | 735 | 1120 |
| | | Evaluation | B | A | B | A | B | B | A | A | B | B | A | B | A | B | B | A |
| | Low temperature stability | | A | A | A | B | B | A | A | C | A | A | A | A | B | B | A | A |

**[Table 2]**

| | | | Comparative Examples | | | |
|---|---|---|---|---|---|---|
| | | | 1 | 2 | 3 | 4 |
| Composition (% by mass) | Component (a) | a-1 | 7.0 | 7.0 | 7.0 | 7.0 |
| | Component (b1) | b1-1 | 10.4 | 10.4 | | 7.8 |
| | Component (b2) | b2-1 | | | | 2.6 |
| | Component (b3) | b3-1 | 2.6 | 2.6 | | 2.6 |
| | Component (c) | c-1 | | | 3.0 | 15 |
| | Component (c') | c'-1 | | 3.0 | | |
| | Purified water | | Balance | Balance | Balance | Balance |
| | Total | | 100 | 100 | 100 | 100 |
| Total content of (a + b) (% by mass) | | | 20 | 20 | 7 | 20 |
| Content of component (a) with respect to total mass of surfactant (% by mass) | | | 35 | 35 | 100 | 35 |
| c / a ratio | | | 0 | 0.43 | 0.43 | 2.1 |
| b1 / (b - b1) ratio | | | 4.0 | 4.0 | - | 1.5 |
| Evaluation | Viscosity | Measured value (mPa·s, 25°C) | 52 | 1227 | - | - |
| | | Evaluation | C | A | - | - |
| | Low temperature stability | | D | D | D | D |

From the results shown in Tables 1 and 2, it was confirmed that the liquid detergents of Examples 1 to 16 to which the present invention was applied had high viscosity and excellent low temperature stability.

On the other hand, the liquid detergent containing no component (c) (Comparative Example 1) was not able to increase the viscosity and had insufficient low temperature stability.

The liquid detergent using the component (c') in place of the component (c) (Comparative Example 2), the liquid detergent containing no component (b) (Comparative Example 3), and the liquid detergent having a c / a ratio of more than 1.5 (Comparative Example 4) did not have sufficient low temperature stability. It should be noted that in Comparative Examples 3 and 4, since the liquid detergent solidified or a large amount of precipitates was produced in the liquid detergent, the viscosity was not measured.

### [Industrial Applicability]

According to the present invention, it is possible to provide a liquid detergent containing an α-SF salt and having an increased viscosity and excellent low temperature stability.

## Claims

1. A liquid detergent comprising:
a surfactant containing an α-sulfo fatty acid alkyl ester salt (a) represented by the following general formula (I) and
at least one type of surfactant (b) selected from sulfonic acid-type anionic surfactants, sulfuric acid ester-type anionic surfactants and nonionic surfactants other than said component (a); and
a water-soluble magnesium salt (c), wherein
the total content of said component (a) and said component (b) is from 8 to 35% by mass with respect to the total mass of the liquid detergent,
the content of said component (a) is 3% by mass or more with respect to the total mass of the liquid detergent,
the content of said component (b) is 5% by mass or more with respect to the total mass of the liquid detergent,
the content of said component (a) is 50% by mass or less with respect to the total mass of the surfactant, and
a mass ratio represented by the formula: (said component (c)) / (said component (a)) is from 0.1 to 1.5,
R¹-CH(SO₃M)-COOR² (I)
wherein R¹ is a hydrocarbon group having 14 to 16 carbon atoms, R² is a hydrocarbon group having 1 to 6 carbon atoms, and M is a counter ion.

2. The liquid detergent according to Claim 1,
wherein said component (b) comprises an alkylbenzene sulfonate (b1), and
a mass ratio represented by the formula: (content of (b1)) / {(content of (b)) - (content of (b1))} is equal to or more than 1.

3. The liquid detergent according to Claim 1 or 2, wherein a viscosity at 25°C is equal to or more than 500 mPa·s.
